# EUROPEAN PATENT SPECIFICATION

(11) **EP 4 516 346 B1**
(45) Date of publication and mention of the grant of the patent: **13.05.2026**
(21) Application number: 24198196.8
(22) Date of filing: 03.09.2024
(51) Int. Cl.: A61M 37/00

(54) **TATTOOING DEVICE**
TÄTOWIERVORRICHTUNG
DISPOSITIF DE TATOUAGE

(30) Priority: 04.09.2023 CN 202322390608 U
(43) Date of publication of application: 05.03.2025
(73) Proprietor: Yue, Heng, Yueyang Hunan 414000 (CN)
(72) Inventor: DAI, Fei, Shijiazhuang, 050000 (CN); YUE, Heng, Yueyang, 414000 (CN)
(74) Representative: Pons IP

(56) References cited:
- US-A1- 2013 226 211
- US-A1- 2019 217 072
- US-A1- 2022 143 378

## Description

### TECHNICAL FIELD

The present invention relates to the technical field of tattooing, in particular to a tattooing device.

### BACKGROUND

Tattoo needle is a special tool for tattoo operation, and its design and material are very important for the effect and safety of tattoo. The working principle of tattoo needle is to evenly puncture the epidermis at a high speed, and implant ink into the dermis under the epidermis. In this way, the human body will try to devour and eliminate foreign invaders when dealing with damaged white blood cells, and the pigment particles are small and easy to be swallowed and can be permanently fixed on the epidermis, forming patterns and characters of various pigment combinations, thus completing tattoos.

In the tattooing needle of the prior art, the needle tip opening of the needle mouth is usually a smooth and flat surface, and the adsorption force between the ink and the needle mouth is not strong, so that when the needle is inserted back and forth from the needle mouth, it is easy to cause ink dripping, ink leakage, ink ejection and the like, which affects the tattooing effect and efficiency; in addition, during the tattooing process of the existing tattooing needle, the needle mouth is mostly inclined downwards, so that the ink flowing out of the needle mouth tends to pollute tattoo machine, and may even cause cross infection of diseases. See, e.g., US2022143378 A1 which discloses a needle module for a tattoo device includes a housing and a needle bundle. The housing comprises a first end for coupling to the tattoo device, a second end comprising a mouth, and a channel between the first and second ends. The needle bundle is moveably mounted in the channel of the housing and is reciprocally moveable in an axial direction between a retracted position and an extended position through the mouth. The mouth comprises at least two jaws. At least one of the jaws is flexible and flareable when radially biased by the needle bundle.

See further US2013226211 A1 which provides a tattoo needle tip equipped with a capillary ink reservoir, a tattoo tube having the said tattoo needle tip, and an assembly of the said tattoo needle tip and a tattoo needle. The tattoo needle tip equipped with a capillary ink reservoir comprises a needle tip body. A tattoo needle passage is provided inside the needle tip body. A needle tip opening is provided at one end of the needle tip body, and the needle tip opening and the tattoo needle passage are connected. The needle tip body is provided with at least one ink reservoir for containing and storing ink. The ink reservoir is provided with at least one ink chamber with capillary action. At least one ink guiding passage connects the capillary ink chamber with the needle tip opening. The tattoo needle tip equipped with an ink reservoir utilizes capillary action to store ink in the ink reservoir, thereby having a big capacity of ink storage and preventing ink leakage from easily occurring.

See finally US2019217072 A1 which discloses a needle assembly for a liquid applicator includes a housing comprising a tubular longitudinal channel and a guide/inner surface. The channel comprises an upper open end and a lower open end. Liquid storage grooves for storing liquid by capillary action extend generally transversely in the guide/inner surface above the lower open end. A needle bundle is mounted in the housing. The needle bundle comprise one or more needles longitudinally reciprocally movable in the channel and being biased against the guide surface, or towards the inner surface, across the grooves such that, during longitudinal reciprocal movement of the needle bundle, the needle bundle maintains contact with the guide surface and the liquid stored in the grooves, or maintains a capillary gap between the needle bundle and the inner surface, for drawing the liquid out of the grooves by capillary action.

Therefore, it is necessary to propose a new tattoo needle. When ink is stored in the needle mouth, the adsorption force between the ink and the needle mouth is greater, thus effectively reducing the occurrence of ink dripping, ink leakage, ink ejection and the like in the tattoo process; moreover, the novel tattooing needle can recycle the ink flowing out of the needle mouth in case of ink leakage during tattooing, so as to avoid ink pollution or cross infection of diseases.

### SUMMARY OF THE INVENTION

The invention is defined by independent claims 1, 12 and subsequent dependent claims 2 - 11, 13 - 15.

### SUMMARY OF THE DISCLOSURE

Embodiments of the invention covered by this patent are defined by the claims below, not this summary. This summary is a high-level overview of various embodiments of the invention and introduces some of the concepts that are further described in the Detailed Description section below. This summary is not intended to identify key or essential features of the claimed subject matter, nor is it intended to be used in isolation to determine the scope of the claimed subject matter. The subject matter should be understood by reference to appropriate portions of the entire specification of this patent, any or all drawings and each claim.

The present invention provides a tattooing device to solve the problems of ink drip, ink leakage, ink ejection and the like in the prior tattooing needle, and that the ink flowing out of the needle mouth tends to cause pollution.

In order to achieve the above object, the present invention adopts the following technical solution:
a tattooing device includes a needle body, wherein the needle body comprises a needle handle, a needle mouth and a needle rod; the needle mouth is installed at one end of the needle handle, and the needle rod is arranged in the needle handle;
a front end of the needle mouth is provided with a needle tip opening; an outer side of the needle tip opening is provided with at least one slit; an edge of one end of the needle handle provided with the needle mouth extends towards the needle mouth and forms a flow blocking part; an interior of the flow blocking part forms an ink receiving groove around the outside of the needle mouth; and a groove bottom of the ink receiving groove is provided with at least one ink return port communicated with the interior of the needle handle.

A tattooing method includes adopting a tattooing device, comprising a needle body, wherein the needle body comprises a needle handle, a needle mouth and a needle rod; the needle mouth is installed at one end of the needle handle, and the needle rod is arranged in the needle handle; a front end of the needle mouth is provided with a needle tip opening; an outer side of the needle tip opening is provided with at least one slit; an edge of one end of the needle handle provided with the needle mouth extends towards the needle mouth and forms a flow blocking part; an interior of the flow blocking part forms an ink receiving groove around the outside of the needle mouth; and a groove bottom of the ink receiving groove is provided with at least one ink return port communicated with the interior of the needle handle;
the method includes the following steps:
Step 1, installing the needle body on a tattoo machine; and
Step 2, sucking the required ink through the needle tip opening of the needle mouth; and
Step 3, starting the tattoo machine to tattoo.

### BRIEF DESCRIPTION OF DRAWINGS

In order to explain the technical scheme of this application more clearly, the drawings needed in the implementation will be briefly introduced below. Obviously, the drawings described below are only some implementations of this application. For those skilled in the art, other drawings can be obtained according to these drawings without creative work..
FIG. 1 is a schematic structural diagram of the present invention.
FIG. 2 is a schematic structural view of the present invention from another angle.
FIG. 3 is a partially enlarged schematic view of A in FIG. 2 according to the present invention.
FIG. 4 is a schematic structural diagram of one side of the needle mouth of the present invention.
FIG. 5 is an exploded view of the present invention.
FIG. 6 is a sectional view of the present invention.
FIG. 7 is a sectional view of the end cover of the present invention.
FIG. 8 is a structural schematic diagram of another embodiment of the present invention.
FIG. 9 is a sectional view of FIG. 8 of the present invention.
FIG. 10 is a schematic structural diagram of a second embodiment of the slit of the present invention.
FIG. 11 is a schematic structural view of a third embodiment of the slit of the present invention.
FIG. 12 is a cross-sectional view of a fourth embodiment of the slit of the present invention.

In the figures: Needle handle (100); Ink receiving groove (101); Cleaning port (102); Ink return port (103); Flow blocking part (104); Needle mouth (200); Needle tip opening (201); Slit (202); Needle rod (300); Push rod (301); Sliding rod (302); Connecting rod (303); Needle (304); End cover (400); Connecting part (401); Clamping groove (402); Slideway (403); Elastic member (500); Corrugated part (501); Guide sleeve (600).

### DESCRIPTION OF EMBODIMENTS

In describing the preferred embodiments, specific terminology will be resorted to for the sake of clarity. It is to be understood that each specific term includes all technical equivalents which operate in a similar manner to accomplish a similar purpose.

While various aspects and features of certain embodiments have been summarized above, the following detailed description illustrates a few exemplary embodiments in further detail to enable one skilled in the art to practice such embodiments. Reference will now be made in detail to embodiments of the inventive concept, examples of which are illustrated in the accompanying drawings. The accompanying drawings are not necessarily drawn to scale. The described examples are provided for illustrative purposes and are not intended to limit the scope of the invention as claimed in independent claims 1, 12 and subsequent dependent claim 2 - 11, 13 - 15. It should be understood, however, that persons having ordinary skill in the art may practice the inventive concept without these specific details.

It will be understood that, although the terms first, second, etc. may be used herein to describe various elements, these elements should not be limited by these terms. These terms are only used to distinguish one element from another. For example, a first attachment could be termed a second attachment, and, similarly, a second attachment could be termed a first attachment, without departing from the scope of the inventive concept.

It will be understood that when an element or layer is referred to as being "on," "coupled to," or "connected to" another element or layer, it can be directly on, directly coupled to or directly connected to the other element or layer, or intervening elements or layers may be present. In contrast, when an element is referred to as being "directly on," "directly coupled to," or "directly connected to" another element or layer, there are no intervening elements or layers present. Like numbers refer to like elements throughout. As used herein, the term "and/or" includes any and all combinations of one or more of the associated listed items.

As used in the description of the inventive concept and the appended claims, the singular forms "a," "an," and "the" are intended to include the plural forms as well, unless the context clearly indicates other.

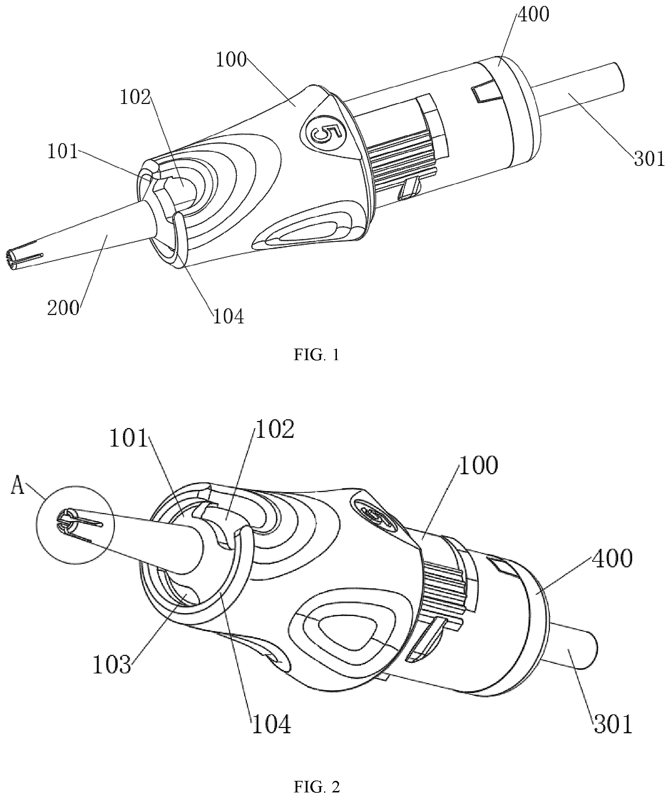

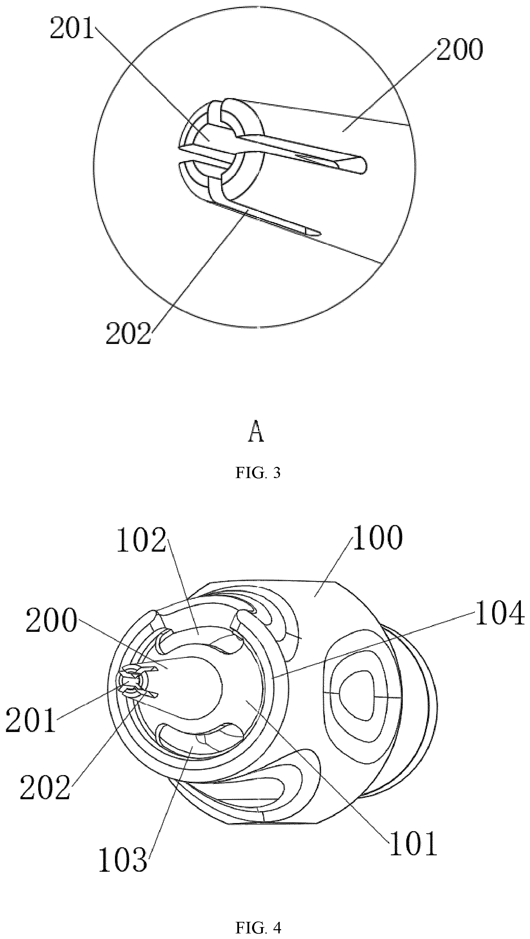

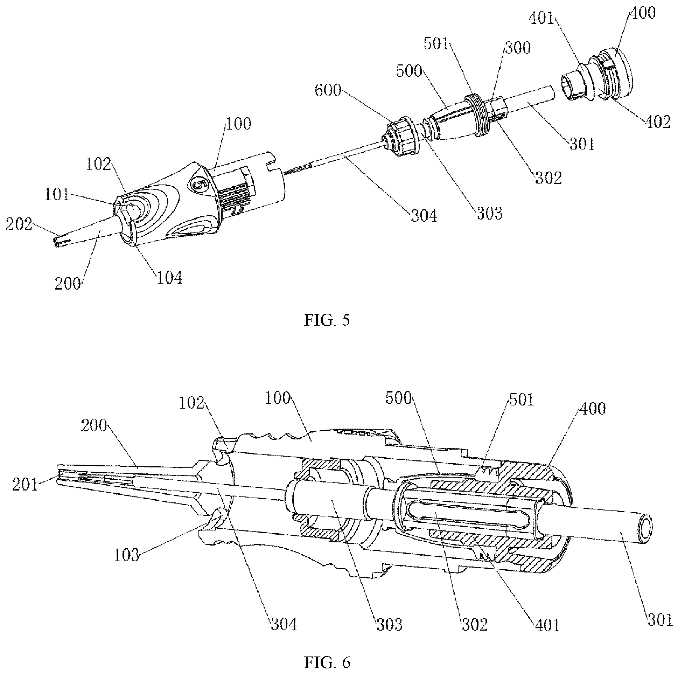

Please refer to FIGS. 1 to 7 and 10 to 12.

The present invention provides a tattooing device, which includes a needle body, wherein the needle body comprises a needle handle 100, a needle mouth 200 and a needle rod 300; the needle mouth 200 is installed at one end of the needle handle 100, and the needle rod 300 is arranged in the needle handle 100; the front end of the needle mouth 200 is provided with a needle tip opening 201; the outer side of the needle tip opening 201 is provided with at least one slit 202; the edge of one end of the needle handle 100 where the needle mouth 200 is installed extends towards the needle mouth 200 and forms a flow blocking part 104; an ink receiving groove 101 around the outside of the needle mouth 200 is formed inside the flow blocking part 104, and the groove bottom of the ink receiving groove 101 is provided with at least one ink return port 103 communicated with the interior of the needle handle 100.

The needle mouth 200 is hollow and communicates with the needle handle 100, and the interior of the needle handle 100 is provided with a guide sleeve 600, and the other end of the needle handle 100 is inserted with an end cover 400; in other embodiments, the end cover 400 is connected by means of, including but not limited to, threaded connection, clamping and the like, and when the end cover 400 and the needle handle 100 are connected by means of threaded connection, the connection between the end cover 400 and the needle handle 100 is closer and the stability is high; while connecting the end cover 400 in a clamping way makes the installation and disassembly of the end cover 400 more convenient.

The inner end of the end cover 400 extends into the needle handle 100 and is set as a connecting part 401, and there is a gap between the connecting part 401 and the needle handle 100. The outer surface of the connecting part 401 is provided with a clamping groove 402 located inside the needle handle 100, and the interior of the connecting part 401 is provided with a slideway 403. The needle rod 300 includes a push rod 301, a sliding rod 302, a connecting rod 303 and a needle 304. The sliding rod 302 is located in the slideway 403 and is connected with the slideway 403 in a sliding way; the push rod 301 is installed at the outer end of the sliding rod 302 and penetrates out of the end cover 400; the connecting rod 303 is installed at the inner end of the sliding rod 302 and penetrates through and is connected with the guide sleeve 600 in a sliding way; the needle 304 is installed at the other end of the connecting rod 303, and the tip of the needle 304 extends into the needle mouth 200; the needle 304 can be connected with the connecting rod 303 in a detachable connection way, including but not limited to threaded connection, clamping and the like, and the needle 304 can be replaced by the detachable connection way.

An elastic member 500 located between the guide sleeve 600 and the end cover 400 is sleeved outside the connecting rod 303, and the other end of the elastic member 500 is sleeved outside the connecting part 401 and embedded in the clamping groove 402.

The outer surface of the elastic member 500 is provided with a corrugated part 501, which is attached to the inner surface of the needle handle 100. The elastic member 500 is made of soft silica gel, and the needle handle 100 is provided with a cleaning port 102 communicating with the ink receiving groove 101, and the cleaning port 102 is communicated with the interior of the needle handle 100.

The elastic member 500 made of silica gel will generate elastic force when it is stretched. By arranging the corrugated part 501, the elastic member 500 can be closely attached to the inner surface of the needle handle 100, so that the elastic member 500 can be fixed between the connecting part 401 and the needle handle 100 through the matching of the corrugated part 501 and the clamping groove 402.

According to the above, the sliding rod 302 can slide in the slideway 403, and the push rod 301 can be connected with the driving part of tattoo machine, so that tattoo machine can push the sliding rod 302 to slide in the slideway 403 through the push rod 301; the sliding rod 302 synchronously drives the connecting rod 303 and the needle 304 to slide, and when the connecting rod 303 slides, the elastic member 500 will be elongated and generate elastic force. Therefore, through the matching of the elastic member 500 and the driving part of the tattoo machine, the needle rod 300 can be pushed to slide back and forth in the needle handle 100, so that the needle 304 can penetrate through the needle tip opening 201 back and forth. During the back and forth movement of the needle rod 300, the guide sleeve 600 can guide and support the movement of the needle rod 300, thus improving the stability of the movement of the needle rod 300.

When the tattooing device is used, the needle tip opening 201 at the needle mouth 200 can be inserted into the ink to dip the ink, so that when the needle 304 penetrates through the needle tip opening 201 back and forth, the ink at the needle tip opening 201 will stick to the needle 304 and then pierce into the human skin.

By arranging the slit 202 at the needle tip opening 201, when the ink is located in the needle tip opening 201, the slit 202 can increase the contact area between the needle mouth 200 and the ink, so that the ink can be well stored in the needle tip opening 201 by using the surface tension of the ink and the adhesion between the liquid and the solid, and it is not easy to cause ink dripping, ink leakage, ink jet and ink shortage during the tattoo process, thus improving the tattoo efficiency.

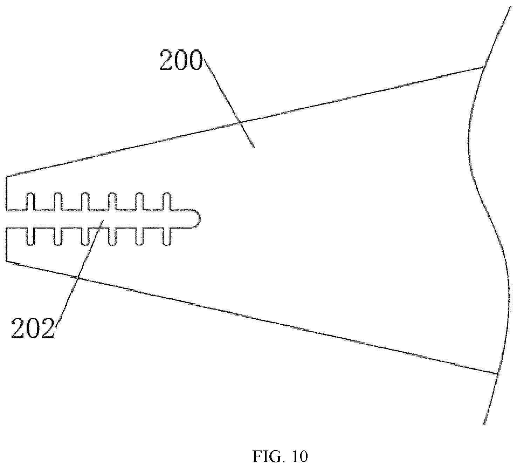

The slit 202 is not limited to the straight shape in this embodiment. In the second application mode of the slit 202, as shown in FIG. 10, a plurality of thin and short branches can be formed on the side of the slit 202, so that the contact area between the ink and the needle mouth 200 can be further increased and the adhesion ability of the ink can be improved.

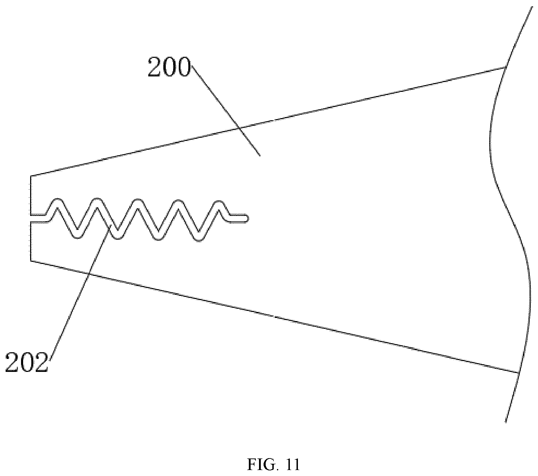

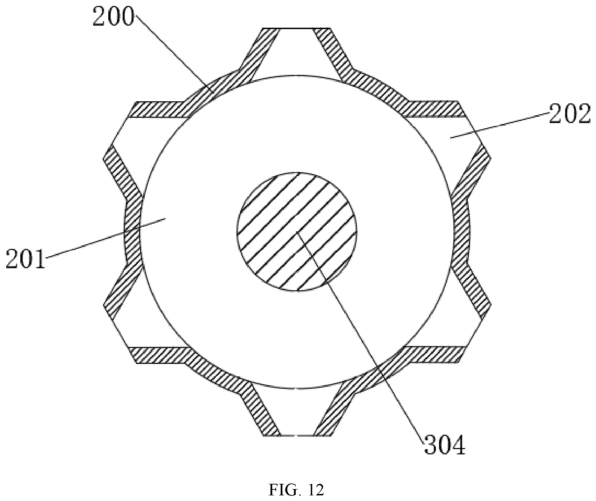

In the third embodiment of the slit 202, as shown in FIG. 11, the slit 202 has a wave shape, and by the wave shape, the contact area between the ink and the needle mouth 200 can be further increased, and the adhesion ability of the ink can be improved;
In the fourth embodiment of the slit 202, as shown in FIG. 12, the cross section of the slit 202 is set in a ladder shape. With this shape, the opening on the outward side of the slit 202 is small and the opening on the inward side is large, which can increase the ink storage capacity of the slit 202, and also improve the contact area between the ink and the needle mouth 200. Moreover, because the opening on the outside of the slit 202 is small, the ink is not easy to drip from the outside of the slit 202.

To sum up, the shape of the slit 202 is not limited to the above-described shape, but can also be any other shape that is conducive to storing ink.

By arranging the flow blocking part 104 and the ink return port 103, the needle handle 100 and the needle mouth 200 are held obliquely upwards during the tattooing process, so that if ink leaks from the needle tip opening 201, the ink will flow downwards along the needle mouth 200, and the flow blocking part 104 will block the ink flowing downwards and collect it in the ink receiving groove 101. The collected ink will enter the needle handle 100 through the ink return port 103 for collection; Therefore, through this structure, it is possible to prevent the leaked ink from polluting the outside of the needle handle 100 and tattoo machine, so as to better achieve hygiene and cleaning and prevent cross infection.

In other embodiments, a sealing ring can be installed between the guide sleeve 600 and the connecting rod 303, so that the guide sleeve 600 can also play a sealing role, so that when the ink is collected in the needle handle 100, the guide sleeve 600 can prevent the ink from flowing to the elastic member 500.

At the same time, the elastic member 500 also plays a second sealing effect, preventing the ink collected in the needle handle 100 from flowing to the end cover 400 and preventing the ink from overflowing from the gap between the end cover 400 and the push rod 301.

By setting the cleaning port 102, it is convenient to clean the ink in the needle handle 100. At the same time, because the cleaning port 102 is also communicated with the ink receiving groove 101, the ink entering the ink receiving groove 101 can also enter the needle handle 100 through the cleaning port 102 for collection. At the same time, the cleaning port 102 also plays a role of ventilation, which can exhaust the air in the needle handle 100 and facilitate the smooth backflow of the ink into the needle handle 100.

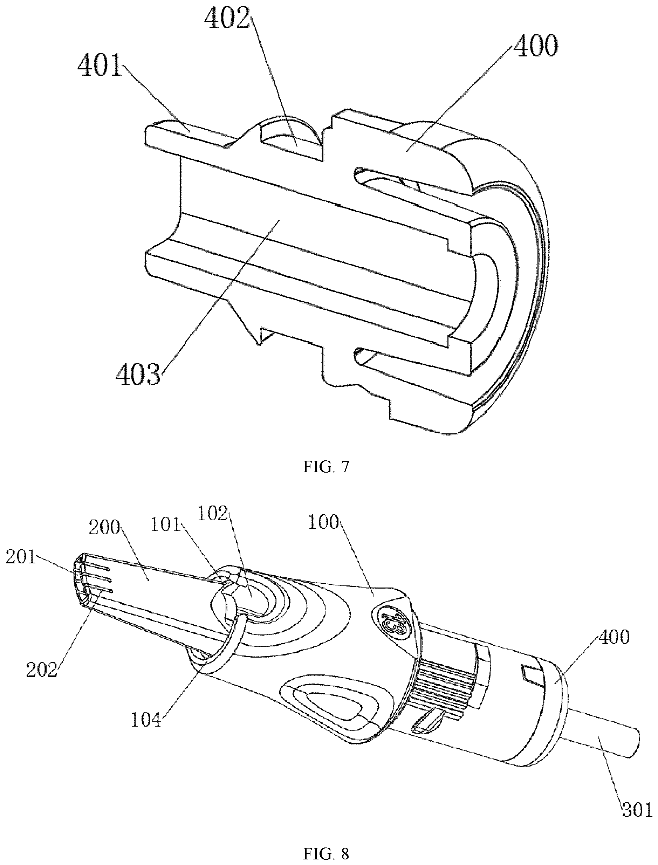

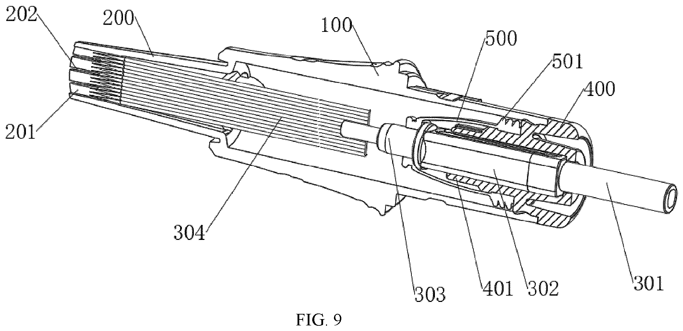

In another embodiment of the tattooing device, as shown in FIGS. 8 and 9, a plurality of needles 304 are arranged in rows, and the needle tip openings 201 are correspondingly arranged in a rectangle, and the rectangular needle tip openings 201 are also correspondingly provided with slits 202, so that the rectangular needle tip openings 201 are also suitable for this ink retaining structure and ink recovery.

The present invention further provides a tattoo method, which comprises adopting a tattooing device comprising a needle body, wherein the needle body comprises a needle handle 100, a needle mouth 200 and a needle rod 300; the needle mouth 200 is installed at one end of the needle handle 100, and the needle rod 300 is arranged in the needle handle 100; the front end of the needle mouth 200 is provided with a needle tip opening 201, and the outer side of the needle tip opening 201 is provided with at least one slit 202; the edge of one end of the needle handle 100 where the needle mouth 200 is installed extends towards the needle mouth 200 and forms a flow blocking part 104, and the interior of the flow blocking part 104 forms an ink receiving groove 101 around the outside of the needle mouth 200; and the groove bottom of the ink receiving groove 101 is provided with at least one ink return port 103 communicating with the interior of the needle handle 100; the method comprises the following steps:
Step 1, installing the needle body on the tattoo machine.
Step 2, sucking the required ink through the needle tip opening 201 of the needle mouth 200; wherein after the needle mouth 200 sucks ink, the ink is kept in the needle tip opening 201 and the slit 202 by the surface tension of the liquid and the adhesion between the liquid and the solid;
Step 3, starting the tattoo machine to tattoo; wherein during tattooing, the needle handle 100 is grasped and the needle mouth 200 is kept in an upward inclined state; in the process of tattooing, the ink flowing from the needle mouth 200 will flow into the ink receiving groove 101 along the outer surface of the needle mouth 200, and the ink in the ink receiving groove 101 will flow into the needle handle 100 through the ink return port 103 for collection.

The technical means disclosed in the scheme of the present invention are not limited to the technical means disclosed in the above embodiments, but also include the technical scheme composed of any combination of the above technical features.

The invention has now been described in detail for the purposes of clarity and understanding. However, those skilled in the art will appreciate that certain changes and modifications may be practiced within the scope of the appended claims.

Conditional language used herein, such as, among others, "can," "could," "might," "may," "e.g.," and the like, unless specifically stated otherwise, or otherwise understood within the context as used, is generally intended to convey that certain examples include, while other examples do not include, certain features, elements, and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular example.

The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. The use of "adapted to" or "configured to" herein is meant as open and inclusive language that does not foreclose devices adapted to or configured to perform additional tasks or steps. Additionally, the use of "based on" is meant to be open and inclusive, in that a process, step, calculation, or other action "based on" one or more recited conditions or values may, in practice, be based on additional conditions or values beyond those recited. Similarly, the use of "based at least in part on" is meant to be open and inclusive, in that a process, step, calculation, or other action "based at least in part on" one or more recited conditions or values may, in practice, be based on additional conditions or values beyond those recited. Headings, lists, and numbering included herein are for ease of explanation only and are not meant to be limiting.

The various features and processes described above may be used independently of one another, or may be combined in various ways. All possible combinations and sub-combinations are intended to fall within the scope of the present disclosure. In addition, certain method or process blocks may be omitted in some implementations. The methods and processes described herein are also not limited to any particular sequence, and the blocks or states relating thereto can be performed in other sequences that are appropriate. For example, described blocks or states may be performed in an order other than that specifically disclosed, or multiple blocks or states may be combined in a single block or state. The example blocks or states may be performed in serial, in parallel, or in some other manner. Blocks or states may be added to or removed from the disclosed examples. Similarly, the example systems and components described herein may be configured differently than described. For example, elements may be added to, removed from, or rearranged compared to the disclosed examples.

## Claims

1. A tattooing device, comprising a needle body, wherein the needle body comprises a needle handle (100), a needle mouth (200) and a needle rod (300); the needle mouth is installed at one end of the needle handle, and the needle rod is arranged in the needle handle; wherein
a front end of the needle mouth is provided with a needle tip opening (201); an outer side of the needle tip opening is provided with at least one slit (202);
an edge of one end of the needle handle provided with the needle mouth extends towards the needle mouth and forms a flow blocking part (104);
an interior of the flow blocking part forms an ink receiving groove (101) around the outside of the needle mouth; and a groove bottom of the ink receiving groove is provided with at least one ink return port (103) communicated with the interior of the needle handle.

2. The tattooing device according to claim 1, wherein the needle handle is provided with a cleaning port (102) communicated with the ink receiving groove, and the cleaning port is communicated with the interior of the needle handle.

3. The tattooing device according to claim 1, wherein the interior of the needle mouth is hollow and communicates with the interior of the needle handle; a guide sleeve (600) is installed inside the needle handle, and an end cover (400) is inserted into the other end of the needle handle; and an inner end of the end cover extends into the needle handle and is set as a connecting part (401).

4. The tattooing device according to claim 3, wherein there is a gap between the connecting part and the needle handle, an outer surface of the connecting part is provided with a clamping groove (402) located inside the needle handle, and the connecting part is provided with a slideway inside.

5. The tattooing device according to claim 4, wherein the needle rod comprises a push rod (301), a sliding rod (302), a connecting rod (303) and a needle (304), and the sliding rod is located in the slideway and connected with the slideway in a sliding way.

6. The tattooing device according to claim 5, wherein the push rod is installed at an outer end of the sliding rod and penetrates out of the end cover.

7. The tattooing device according to claim 6, wherein the connecting rod is installed at an inner end of the sliding rod, and the connecting rod penetrates through the guide sleeve and is slidably connected with the guide sleeve.

8. The tattooing device according to claim 7, wherein the needle is installed at the other end of the connecting rod, and a tip of the needle extends into the needle mouth.

9. The tattooing device according to claim 7, wherein an elastic member located between the guide sleeve and the end cover is sleeved outside the connecting rod, and the other end of the elastic member (500) is sleeved outside the connecting part and embedded in the clamping groove.

10. The tattooing device according to claim 9, wherein an outer surface of the elastic member is provided with a corrugated part (501), and the corrugated part is attached to an inner surface of the needle handle.

11. The tattooing device according to claim 9, wherein the elastic member is made of soft silica gel.

12. A tattooing method, which comprises adopting a tattooing device, comprising a needle body, wherein the needle body comprises a needle handle (100), a needle mouth (200) and a needle rod (300);
the needle mouth is installed at one end of the needle handle, and the needle rod is arranged in the needle handle; a front end of the needle mouth is provided with a needle tip opening (201);
an outer side of the needle tip opening is provided with at least one slit (202);
an edge of one end of the needle handle provided with the needle mouth extends towards the needle mouth and forms a flow blocking part (104);
an interior of the flow blocking part forms an ink receiving groove (101) around the outside of the needle mouth; and a groove bottom of the ink receiving groove is provided with at least one ink return port (103) communicated with the interior of the needle handle;
the method comprises the following steps:
Step 1, installing the needle body on a tattoo machine; and
Step 2, sucking the required ink through the needle tip opening of the needle mouth; and
Step 3, starting the tattoo machine to tattoo.

13. The tattooing method according to claim 12, wherein after the needle mouth sucks ink, the ink is kept in the needle tip opening and the slit by the surface tension of liquid and the adhesion between liquid and solid.

14. The tattooing method according to claim 12, wherein during tattooing, the needle handle is grasped and the needle mouth is kept in an upward inclined state.

15. The tattooing method according to claim 14, wherein during tattooing , the ink flowing out from the needle mouth will flow into the ink receiving groove along the outer surface of the needle mouth, and the ink in the ink receiving groove will flow into the needle handle through the ink return port for collection.

## Patentansprüche

1. Tätowiervorrichtung, umfassend einen Nadelkörper, wobei der Nadelkörper einen Nadelhalter (100), eine Nadeldüse (200) sowie eine Nadelstange (300) umfasst, die Nadeldüse an einem Ende des Nadelhalters montiert ist und die Nadelstange im Nadelhalter angeordnet ist, **dadurch gekennzeichnet, dass**:
am vordersten Ende der Nadeldüse eine Nadelspitzenöffnung (201) ausgebildet ist, an der Außenfläche der Nadelspitzenöffnung mindestens ein Schlitz (202) ausgebildet ist, der Rand des Nadelhalters an dem mit der Nadeldüse versehenen Ende sich in Richtung der Nadeldüse erstreckt und einen Rückhalteabschnitt (104) bildet, die Innenseite des Rückhalteabschnitts eine die Außenseite der Nadeldüse umgebende Tintensammelnut (101) bildet und im Nutboden der Tintensammelnut mindestens eine mit dem Inneren des Nadelhalters in Verbindung stehende Tintenrücklauföffnung (103) ausgebildet ist.

2. Tätowiervorrichtung nach Anspruch 1, **dadurch gekennzeichnet, dass** am Nadelhalter eine mit der Tintensammelnut in Verbindung stehende Reinigungsöffnung (102) ausgebildet ist und die Reinigungsöffnung mit dem Inneren des Nadelhalters in Verbindung steht.

3. Tätowiervorrichtung nach Anspruch 1, **dadurch gekennzeichnet, dass** die Nadeldüse innen hohl und mit dem Inneren des Nadelhalters verbunden ist, im Inneren des Nadelhalters eine Führungshülse (600) montiert ist und in das andere Ende des Nadelhalters eine Endkappe (400) eingesteckt ist, wobei das innere Ende der Endkappe sich in den Nadelhalter hinein erstreckt und als Verbindungsabschnitt (401) ausgebildet ist.

4. Tätowiervorrichtung nach Anspruch 3, **dadurch gekennzeichnet, dass** zwischen dem Verbindungsabschnitt und dem Nadelhalter ein Zwischenraum vorhanden ist, an der Außenfläche des Verbindungsabschnitts eine im Inneren des Nadelhalters befindliche Rastnut (402) ausgebildet ist und im Inneren des Verbindungsabschnitts ein Gleitkanal ausgebildet ist.

5. Tätowiervorrichtung nach Anspruch 4, **dadurch gekennzeichnet, dass** die Nadelstange eine Schubstange (301), eine Gleitstange (302), eine Verbindungsstange (303) sowie eine Nadelspitze (304) umfasst und die Gleitstange im Gleitkanal angeordnet und mit dem Gleitkanal gleitend verbunden ist.

6. Tätowiervorrichtung nach Anspruch 5, **dadurch gekennzeichnet, dass** die Schubstange am äußeren Ende der Gleitstange montiert ist und nach außen durch die Endkappe hindurchragt.

7. Tätowiervorrichtung nach Anspruch 6, **dadurch gekennzeichnet, dass** die Verbindungsstange am inneren Ende der Gleitstange montiert ist und die Verbindungsstange durch die Führungshülse hindurchragt und mit der Führungshülse gleitend verbunden ist.

8. Tätowiervorrichtung nach Anspruch 7, **dadurch gekennzeichnet, dass** die Nadelspitze am anderen Ende der Verbindungsstange montiert ist und das spitze Ende der Nadelspitze sich in das Innere der Nadeldüse hinein erstreckt.

9. Tätowiervorrichtung nach Anspruch 7, **dadurch gekennzeichnet, dass** auf der Außenseite der Verbindungsstange ein zwischen der Führungshülse und der Endkappe befindliches elastisches Element (500) aufgesteckt ist und das andere Ende des elastischen Elements auf der Außenseite des Verbindungsabschnitts aufgesteckt ist und in die Rastnut eingreift.

10. Tätowiervorrichtung nach Anspruch 9, **dadurch gekennzeichnet, dass** an der Außenfläche des elastischen Elements ein Wellenabschnitt (501) vorgesehen ist und der Wellenabschnitt an der Innenfläche des Nadelhalters anliegt.

11. Tätowiervorrichtung nach Anspruch 9, **dadurch gekennzeichnet, dass** das elastische Element aus weichem Silikonmaterial besteht.

12. Tätowierverfahren, wobei das Verfahren die Verwendung einer Tätowiervorrichtung umfasst, wobei die Vorrichtung einen Nadelkörper umfasst, der Nadelkörper einen Nadelhalter (100), eine Nadeldüse (200) sowie eine Nadelstange (300) umfasst, die Nadeldüse an einem Ende des Nadelhalters montiert ist, die Nadelstange im Nadelhalter angeordnet ist, am vordersten Ende der Nadeldüse eine Nadelspitzenöffnung (201) ausgebildet ist, an der Außenfläche der Nadelspitzenöffnung mindestens ein Schlitz (202) ausgebildet ist, der Rand des Nadelhalters an dem mit der Nadeldüse versehenen Ende sich in Richtung der Nadeldüse erstreckt und einen Rückhalteabschnitt (104) bildet, die Innenseite des Rückhalteabschnitts eine die Außenseite der Nadeldüse umgebende Tintensammelnut (101) bildet und im Nutboden der Tintensammelnut mindestens eine mit dem Inneren des Nadelhalters in Verbindung stehende Tintenrücklauföffnung (103) ausgebildet ist; und
wobei das Verfahren die folgenden Schritte umfasst:
Schritt 1: Montieren des Nadelkörpers auf einer Tätowiermaschine; und
Schritt 2: Aufnehmen der benötigten Tinte durch die Nadelspitzenöffnung der Nadeldüse; und
Schritt 3: Starten der Tätowiermaschine zur Durchführung des Tätowiervorgangs.

13. Tätowierverfahren nach Anspruch 12, **dadurch gekennzeichnet, dass** nach dem Aufnehmen der Tinte durch die Nadeldüse die Tinte mittels der Oberflächenspannung der Flüssigkeit und der Adhäsionskraft zwischen Flüssigkeit und Festkörper in der Nadelspitzenöffnung und dem Schlitz gehalten wird.

14. Tätowierverfahren nach Anspruch 12, **dadurch gekennzeichnet, dass** beim Tätowieren der Nadelhalter gegriffen wird und die Nadeldüse in einem nach oben geneigten Zustand gehalten wird.

15. Tätowierverfahren nach Anspruch 14, **dadurch gekennzeichnet, dass** während des Tätowiervorgangs die aus der Nadeldüse austretende Tinte entlang der Außenfläche der Nadeldüse in die Tintensammelnut fließt und die Tinte in der Tintensammelnut durch die Tintenrücklauföffnung in das Innere des Nadelhalters fließt und dort gesammelt wird.

## Revendications

1. Dispositif de maquillage permanent, **caractérisé en ce qu'**il comprend : un corps d'aiguille, ce corps d'aiguille incluant un manche (100), une pointe d'aiguille (200) et une tige d'aiguille (300), la pointe d'aiguille étant montée à une extrémité du manche et la tige d'aiguille étant disposée à l'intérieur du manche, **caractérisé en ce que** :
L'extrémité de la pointe d'aiguille présente une ouverture (201) ; la face externe de cette ouverture comporte au moins une fente (202) ; le bord du manche de l'aiguille, à l'extrémité où est fixée la pointe, se prolonge vers celle-ci et forme un dispositif d'arrêt d'écoulement (104) ; la face interne de ce dispositif forme une rainure de collecte d'encre (101) entourant la face externe de la pointe d'aiguille ; le fond de cette rainure comporte au moins un orifice de retour d'encre (103) communiquant avec l'intérieur du manche de l'aiguille.

2. Dispositif de maquillage permanent selon la revendication 1, **caractérisé en ce que** : un orifice de nettoyage (102) communiquant avec la rainure de collecte d'encre est prévu sur le manche de l'aiguille, et l'orifice de nettoyage communique avec l'intérieur du manche de l'aiguille.

3. Dispositif de maquillage permanent selon la revendication 1, **caractérisé en ce que** : l'intérieur de la point de l'aiguille est creuse et communique avec l'intérieur du manche ; un manchon de guidage (600) est installé à l'intérieur du manche ; un embout (400) est inséré à l'autre extrémité du manche ; l'extrémité intérieure de l'embout s'étend dans le manche et constitue une pièce de liaison (401).

4. Dispositif de maquillage permanent selon la revendication 3, **caractérisé en ce que** : un espace existe entre la pièce de liaison et le manche ; une rainure (402) est formée à l'intérieur du manche sur la surface extérieure de ladite pièce de liaison ; et un rail de guidage est formé à l'intérieur de la partie de liaison.

5. Dispositif de maquillage permanent selon la revendication 4, **caractérisé en ce que** : la tige d'aiguille comprend une tige de poussée (301), une tige de guidage (302), une tige de liaison (303) et une extrémité de la tige d'aiguille (304) ; la tige de guidage est située à l'intérieur du rail de guidage et reliée à celui-ci de manière coulissante.

6. Dispositif de maquillage permanent selon la revendication 5, **caractérisé en ce que** : la tige de poussée est installée à l'extrémité extérieure de la tige de guidage et se prolonge vers l'extérieur à travers un embout.

7. Dispositif de maquillage permanent selon la revendication 6, **caractérisé en ce que** : la tige de liaison est installée à l'extrémité intérieure de la tige de guidage ; elle traverse le manchon de guidage et y coulisse.

8. Dispositif de maquillage permanent selon la revendication 7, **caractérisé en ce que** : l'aiguille est installée à l'autre extrémité de la tige de liaison, et que son extrémité s'étend à l'intérieur de la pointe d'aiguille.

9. Dispositif de maquillage permanent selon la revendication 7, **caractérisé en ce que** : un élément élastique (500) est gainé à l'extérieur de la tige de liaison, entre le manchon de guidage et l'embout, et que l'autre extrémité de cet élément élastique est gainée à l'extérieur de la partie de liaison et encastrée dans une rainure.

10. Dispositif de maquillage permanent selon la revendication 9, **caractérisé en ce que** : la surface extérieure de l'élément élastique présente une partie ondulée (501), cette partie ondulée étant en contact avec la surface intérieure du manche de l'aiguille.

11. Dispositif de maquillage permanent selon la revendication 9, **caractérisé en ce que** : l'élément élastique est en silicone souple.

12. Procédé de maquillage permanent, comprenant l'utilisation d'un dispositif de maquillage permanent, ce dispositif comprenant un corps d'aiguille, ce corps d'aiguille comprenant un manche (100), une pointe d'aiguille (200) et une tige d'aiguille (300). La pointe d'aiguille est montée à une extrémité du manche, la tige d'aiguille est disposée à l'intérieur du manche, l'extrémité avant de la pointe d'aiguille présente une ouverture (201), le côté extérieur de l'ouverture présente au moins une fente (202), le bord du manche, à l'extrémité où est montée la pointe d'aiguille, s'étend vers celle-ci et forme un dispositif d'arrêt d'écoulement (104), le côté intérieur du dispositif d'arrêt d'écoulement forme une rainure de collecte d'encre (101) entourant le côté extérieur de la pointe d'aiguille, et le fond de la rainure de collecte d'encre présente au moins un orifice de retour d'encre (103) communiquant avec l'intérieur du manche.
Le procédé comprenant les étapes suivantes :
Étape 1 : monter le corps de l'aiguille sur une machine à tatouer ;
Étape 2 : aspirer de l'encre requise à travers la pointe de l'aiguille ;
Étape 3 : mettre en marche la machine à tatouer pour réaliser le tatouage.

13. Procédé de maquillage selon la revendication 12, **caractérisé en ce que** : après l'aspiration de l'encre par la pointe de l'aiguille, celle-ci est retenue dans la pointe et la fente grâce à la tension superficielle du liquide et à l'adhérence entre le liquide et le solide.

14. Procédé de maquillage selon la revendication 12, **caractérisé en ce que** : pendant le processus de maquillage permanent, le manche de l'aiguille est tenu et la pointe de l'aiguille est inclinée vers le haut.

15. Procédé de maquillage permanent selon la revendication 14, **caractérisé en ce que** : pendant le processus de maquillage permanent, l'encre s'écoulant de la pointe de l'aiguille longe la surface extérieure de celle-ci et pénètre dans la rainure de collecte d'encre, puis s'écoule dans le manche de l'aiguille pour être recueillie par l'orifice de retour d'encre.
